# EUROPEAN PATENT APPLICATION

(11) **EP 4 506 951 A1**
(43) Date of publication of application: **12.02.2025**
(21) Application number: 23189955.0
(22) Date of filing: 07.08.2023
(51) Int. Cl.: G16H 20/40, A61B 34/20, A61B 34/35, G06F 3/01, G06F 9/451, G16H 40/67, H04N 7/18, H04N 23/60, A61B 17/00

(54) **REMOTE MONITORING OF SURGICAL PROCEDURE**

(71) Applicant: Koninklijke Philips N.V., 5656 AG Eindhoven (NL)
(72) Inventor: KUHLMANN, Daan, 5656 AG Eindhoven (NL); BUIL, Vincentius Paulus, 5656 AG Eindhoven (NL); WIJN, Victor, 5656 AG Eindhoven (NL); BALICKI, Marcin Arkadiusz, 5656 AG Eindhoven (NL); VAN OORDE-GRAINGER, Shaun, 5656 AG Eindhoven (NL); BOSMAN, Reinoud, 5656 AG Eindhoven (NL)
(74) Representative: Philips Intellectual Property & Standards

(57) **Abstract**

In a surgical workspace, a plurality of monitoring devices 10-30 may be provided to enable a surgical procedure to be remotely monitored using a remote monitoring system 200. A monitoring control system 100 and a computer-implemented monitoring control method are provided which are configured to access monitoring data 40, analyse the monitoring data to determine a current state of the surgical procedure, determine one or more observability requirements for the current state of the surgical procedure, and based thereon, select a subset of the plurality of monitoring devices to be perceptual prioritized by the remote monitoring system. This selection is then conveyed the remote monitoring system 200 via selection data 162. Thereby, the remote monitoring system may be controlled to enable optimal remote monitoring of the surgical procedure.

## Description

### FIELD OF THE INVENTION

The invention relates to a monitoring control system and computer-implemented method for use in a surgical procedure which is remotely monitorable. The invention further relates to a remote monitoring system comprising the monitoring control system. The invention further relates to a computer-readable medium comprising instructions for a computer program, the computer program comprising instructions to cause a processor system to perform the computer-implemented method.

### BACKGROUND OF THE INVENTION

While traditionally surgical procedures are performed manually, e.g., by a surgeon, increasingly, surgical robots are used to assist in surgical procedures or even to entirely carry out certain surgical procedures. While such surgical robots typically remain under the control of an operator, e.g., the surgeon, surgical robots may provide improved dexterity, acuity and other advantages offered by mechanization and digitization.

One of such advantages is that the operator of the surgical robot may be remotely located from the surgical workspace containing the patient and the surgical robot. To nevertheless be able to control the surgical robot, the operator of the surgical robot may make use of a remote control system, such as a workstation with a graphical user interface. Such remote-controlled robotic-assisted surgery may also be referred to as telesurgery and may help patients gain better access to quality surgical care as the geographical barriers between a patient and the operator may be eliminated. This in turn may improve the quality of surgical care in medically underserved areas and/or may eliminate long distance travel.

To enable the operator to control the surgical robot, the remote control system may provide access to one or more camera feeds of cameras located in the surgical workspace. Through the camera feeds, the operator may monitor the current state of the patient, the surgical robot, and the overall surgical procedure and appropriately control the surgical robot. In addition to cameras, the operator may also have access to the monitoring data of other monitoring devices, for example to an audio feed of a microphone in the surgical workspace or to medical data provided by medical devices in the workspace, such as a patient monitor or an interventional imaging system. Ideally, through such monitoring devices, the remote operator is enabled to perceive the situation in the surgical workspace in a similar way as if the operator were locally present in the surgical workspace.

Nevertheless, such monitoring devices may fail to give the operator the same situational awareness as if he/she were locally present in the surgical workspace. For example, a single camera feed may be insufficient to be able to observe both the surgery and local staff, such as a surgical assistant, in the surgical workspace. This problem may be addressed in part by providing more monitoring devices, e.g., multiple camera feeds with different field of views, multiple audio feeds, more medical data from medical devices, etc.

While the above refers to a specific surgical procedure in which a surgical robot is used, the problem of insufficient situational awareness is even more critical and also applies to situations in which a remote monitoring system is provided not necessarily only to control a surgical robot but to allow a remote user to (only) monitor the surgical procedure, e.g., for the remote user to act as a second pair of expert eyes for a member of staff which is locally present in the surgical workspace.

### SUMMARY OF THE INVENTION

It is therefore desirable to enable a remote user to obtain an improved situational awareness when remotely monitoring a surgical procedure. It is also desirable to enable that the sheer amount of monitoring data provided by the monitoring devices does not overwhelm the operator. Indeed, it may be difficult for the remote operator to obtain good situational awareness when being provided with the monitoring data of a plurality of monitoring devices in case the number of monitoring devices is relatively high.

In a first aspect of the invention, a monitoring control system is provided for use in a surgical procedure, wherein a plurality of monitoring devices is provided to monitor the surgical procedure, wherein a remote monitoring system is configured to render monitoring data provided by the plurality of monitoring devices to enable remote monitoring of the surgical procedure, wherein the monitoring control system comprises:
- an input interface for accessing the monitoring data provided by the plurality of monitoring devices;
- an output interface to the remote monitoring system;
- a processor subsystem configured to, during the surgical procedure:
- analyse the monitoring data to determine a current state of the surgical procedure;
- determine one or more observability requirements for the current state of the surgical procedure based on observability data which is indicative of observability requirements for different states of the surgical procedure;
- based on the one or more observability requirements, select a subset of the plurality of monitoring devices to be perceptual prioritized by the remote monitoring system; and
- send selection data indicative of said selection via the output interface to the remote monitoring system.

In a further aspect of the invention, a computer-implemented method is provided for being performed during a surgical procedure, wherein a plurality of monitoring devices is provided to monitor the surgical procedure, wherein a remote monitoring system is configured to render monitoring data provided by the plurality of monitoring devices to enable remote monitoring of the surgical procedure, wherein the method comprises:
- accessing the monitoring data provided by the plurality of monitoring devices;
- analysing the monitoring data to determine a current state of the surgical procedure;
- determining one or more observability requirements for the current state of the surgical procedure based on observability data which is indicative of observability requirements for different states of the surgical procedure;
- based on the one or more observability requirements, selecting a subset of the plurality of monitoring devices to be perceptual prioritized by the remote monitoring system; and
- sending selection data indicative of said selection via the output interface to the remote monitoring system.

In a further aspect of the invention, a transitory or non-transitory computer-readable medium is provided, wherein the computer-readable medium comprises data representing a computer program, the computer program comprising instructions for causing a processor system to perform any of the described computer-implement methods.

The above measures involve remote monitoring of a surgical procedure. The surgical procedure may be a minimally invasive procedure but also any other type of invasive procedure. To enable remote monitoring, a plurality of monitoring devices may be provided in a surgical workspace in which the surgical procedure is carried out, for example in an operating room, emergency room, examination room, catheterization laboratory, etc. A remote monitoring system may be provided remotely from the surgical workplace, for example in a different room, different building, or entirely different location (e.g., different city or country). The remote monitoring system may nevertheless have access to the monitoring data generated by the monitoring devices, for example via a network, and may render the monitoring data to a remote user, for example visually and/or auditory. It is noted that remote monitoring in as far as described in this paragraph may be known per se.

The above measures further involve providing a monitoring control system which may be configured to be used with the remote monitoring system, in that the monitoring control system may control certain aspects of the operation of the remote monitoring system. This may be explained as follows. The monitoring control system may access the monitoring data generated by the monitoring devices, e.g., via a wired or wireless connection. For example, the monitoring control system may access camera feeds of cameras and audio feeds of microphones installed in the surgical workplace, and may in some examples also access medical data which is acquired and/or generated by medical devices in the surgical workplace and which medical data may also allow monitoring of the surgical procedure. The monitoring control system may analyse the monitoring data to determine a current state of the surgical procedure. Here, the term `current state' may refer a characterization of the progress and/or status of the surgical procedure, or an identification of a deviation of status or progress of the procedure with respect to a planned surgical procedure, at a given point in time. The current state may for example be described by details such as: which steps of the procedure have been completed, which issues have arisen, and/or what actions are or have been taken by medical staff. In a specific example, the current state as determined by the monitoring control system may indicate which surgical step is being carried-out, or that there is a pause or timeout, or that there is an interoperative consultation during the surgery, etc.

Having characterized the current state of the surgical procedure, the monitoring control system may determine one or more observability requirements for the current state. Such observability requirements, which may also be referred to as 'observability objectives', may characterize how the current state may be optimally observed, in particular if determined surgical procedural objectives (which may be one or a successive and/or cumulative objectives over a surgical process), defined by e.g., surgical procedural parameters, are to be reached. To determine the observability requirement(s) for the current state, observability data or parameters may be accessed which is indicative of or include observability requirements for different states of the surgical procedure. For example, the observability data or parameters may define that in case of an interoperative consultation, an audio feed which captures member(s) of staff is to be provided, or that in a surgical step, a camera feed is to be provided which provides an obstruction-free view of the surgical entry site. Such observability requirements may for example be obtained from a set of predetermined rules which map a respective state to a respective set of observability requirements, or may be generated by a machine learning model which has been trained to provide such observability requirements from a description of a state of a surgical procedure as input. The term `observability data' may thus include a rule-based system, a trained machine learning model and other examples. Based on the one or more observability requirements, the monitoring control system may select a subset of the plurality of monitoring devices to be perceptual prioritized in the rendering by the remote monitoring system. In particular, the subset may be selected to as much as possible meet the observability requirements. For example, if the observability requirements indicate that an audio recording which captures the member(s) of staff is to be provided, the monitoring control system may select a microphone worn by the member(s) of staff for rendering. Another example is that if the observability requirements indicate that a camera recording is to be provided which provides an obstruction-free view of the surgical entry site, the monitoring control system may select a ceiling-mounted camera which is located above the patient and which provides the aforementioned obstruction-free view.

The monitoring control system may provide selection data indicative of the selection to the remote monitoring system. In turn, the remote monitoring system may be configured to, based on the selection data, perceptually prioritize the subset of monitoring devices in the rendering. Such perceptual prioritization may for example involve the remote monitoring system only selecting the monitoring data of the subset of monitoring devices for rendering, or highlighting the monitoring data of the subset of monitoring devices, for example by changing a size of a camera feed or adjusting the volume of an audio feed.

The above measures have the effect that the remote monitoring system may be controlled to enable optimal remote monitoring of the surgical procedure. In particular, the remote user may be perceptually directed towards the monitoring data which is deemed to be of relevance to observe the current state of the surgical procedure and may be less, or not all, distracted by other monitoring data. Compared to the situation in which all monitoring data is rendered simultaneously, the user may be less overwhelmed and may be able to better focus on the monitoring data of relevance. It may also not be needed for the remote user having to manually select monitoring devices for rendering, for example in cases where it is not practical to render all of the monitoring data simultaneously. Such manual selection may otherwise require effort and distract from the surgical procedure. Advantageously, the remote user's situational awareness may be improved. If the remote user is involved in the surgical procedure, e.g., by acting as a pair of expert eyes or by remotely controlling aspects of the surgical procedure, this may contribute to the success of the surgical procedure.

Optionally, the processor subsystem is configured for, and the computer-implemented method further comprises:
- accessing surgical planning data which is indicative of a planned surgical procedure to be conducted;
- determining the current state of the surgical procedure based on a comparison to the planned surgical procedure

A computer-readable version of the planned surgical procedure may be available and may be accessed and used to determine the current state of the surgical procedure. Advantageously, the planned surgical procedure may give a frame of reference which may allow the monitoring control system to better identify the current state of the surgical procedure. For example, from the surgical planning data, it may be known which type of surgery is to be performed, which type of surgical steps are to be taken, the order of steps, etc. The progress and/or status of the surgical procedure at a given point in time may then be determined in relation to the planned surgical procedure.

Optionally, the processor subsystem is configured for, and the computer-implemented method further comprises:
- based on the comparison, determining whether the current state represents a deviation from the planned surgical procedure; and
- if the deviation is determined, selecting the subset to include at least one monitoring device through which the deviation is remotely observable.

Deviations from the surgical procedure, intended or otherwise, may be particularly relevant when monitoring the surgical procedure. For example, it may be needed for the remote user to react to the deviation or to assist local staff in addressing the cause of the deviation. As such, it may be of particular relevance for the remote user to be able to accurately observe the deviation. To assist the remote user in doing so, the monitoring control system may determine whether such a deviation occurred, and if so, select the subset to include one or more monitoring device through which the situation can be remotely observed. For example, if an instrument has a malfunction during the surgical procedure, a camera view of the instrument may be selected for rendering.

Optionally, the processor subsystem is configured for, and the computer-implemented method further comprises:
- analysing the monitoring data to identify an entity which has caused, contributed to, or is otherwise associated with the deviation; and
- selecting the subset to include at least one monitoring device through which the entity is remotely observable.

The monitoring control system may determine that an entity is of relevance in the assessment of a situation concerning a deviation from the planned surgical procedure. For example, in case of an unplanned interoperative consultation between local members of staff, monitoring devices capturing the member of staffs may be selected to enable the remote user to follow the interoperative consultation.

For example, the entity may be at least one of:
- a surgical target of the surgical procedure,
- a surgical access point,
- an instrument used in the surgical procedure,
- a member of staff attending the surgical procedure,
- a patient;
- a surgical robot, and
- a medical device used during the surgical procedure.

Optionally, the plurality of monitoring devices comprises at least one of: an audio-visual recording device, such as a camera or a microphone, a sensor or component configured to monitor an operational state of an instrument or a surgical robot, and a medical device, such as a patient monitor or an interventional imaging system. All of the aforementioned monitoring devices may provide data which may be used to monitor the surgical procedure, for example to visually and/or auditory perceive the surgical workplace, the patient, staff, etc., or to perceive the medical state of the patient, or to perceive the operational state of an instrument or surgical robot, etc.

Optionally, the processor subsystem is configured for, and the computer-implemented method further comprises:
- based on the current state of the surgical procedure, predicting a following step of the surgical procedure; and
- based on the observability data, selecting the subset to include at least one monitoring device through which the following step is remotely observable.

Optionally, the processor subsystem is configured for, and the computer-implemented method further comprises, determining the current state of the surgical procedure by determining a current step in the surgical procedure. The monitoring control system may determine a current step in the surgical procedure, which may then serve as a characterization of the current state of the surgical procedure.

Optionally, the processor subsystem is configured for, and the computer-implemented method further comprises, determining the current step in the surgical procedure by identifying a surgical event in the monitoring data. The current step may be determined by analysing the monitoring data, for example using computer vision or computer audition or in general by using a data processing or machine learning technique. In particular, surgical event(s) may be identified in the monitoring data, which surgical event(s) may be indicative of the current step in the surgical procedure.

Optionally, the remote monitoring system is configured to enable a remote user of the remote monitoring system to communicate with a member of staff attending the surgical procedure, wherein the processor subsystem is configured for, and the computer-implemented method further comprises, selecting the subset to include at least one monitoring device through which the member of surgical staff is perceptually perceivable, for example visually and/or auditory. It may be of particular relevance for the remote user to be able to communicate with local members of staff, especially if the remote user takes an active role in the surgical procedure. An example of such an active role is if the remote user remotely operates a surgical robot during the surgical procedure. In case a deviation occurs in the surgical procedure, there is a chance of miscommunication, which in turn may lead to errors and may potentially endanger the patient. The monitoring control system may support optimal communication between the remote user and local member(s) of staff by selecting the subset of monitoring devices to include for example a microphone and camera which captures the member(s) of staff.

Optionally, the remote monitoring system comprises or is connected to an eye-tracker to eye-track a remote user of the remote monitoring system, and wherein the processor subsystem is configured for, and the computer-implemented method further comprises:
- accessing eye tracking data from the eye-tracker to determine which monitoring data is observed by the remote user; and
- adjusting the selection data based on the eye tracking data.

Based on the eye tracking data, the monitoring system may learn whether the selection of monitoring device(s) is relevant for the remote user and adjust this selection, if needed, for example by removing monitoring devices from the selected subset which provide monitoring data which is infrequently viewed by the remote user, and/or by adding monitoring devices to the selected subset which are similar in type or characteristic to monitoring devices which are frequently viewed by the remote user.

Optionally, the processor subsystem is configured for, and the computer-implemented method further comprises, adjusting a configuration of a monitoring device and/or a medical device used in the surgery procedure by sending configuration data to the monitoring device and/or the medical device. This way, the monitoring control system may retune monitoring devices and medical devices, for example to allow better observability of the current step or future steps of the surgical procedure.

Optionally, the remote monitoring system is configured to perceptually prioritize the monitoring data of the subset of the plurality of monitoring devices by selectively rendering or perceptually highlighting the monitoring data of the subset of the plurality of monitoring devices.

Optionally, a surgical robot performs one or more surgical steps during the surgical procedure, and the remote monitoring system is a remote control system to remotely control the surgical robot. The monitoring control system may be used during a robotic-assisted remote surgery to provide a remote operator controlling the surgical robot with a better situational awareness, which may enhance the control of the robot.

Optionally, the system is a subsystem of the remote monitoring system. In other words, the remote monitoring system, which may in some examples be a remote control system, may comprise the monitoring control system as a subsystem.

In a further aspect of the invention, a monitoring control system is provided for use in a surgical procedure, wherein a plurality of monitoring devices is provided to monitor the surgical procedure, wherein a remote monitoring system is configured to render monitoring data provided by the plurality of monitoring devices to enable remote monitoring of the surgical procedure, wherein the monitoring control system comprises:
an input interface for accessing the monitoring data provided by the plurality of monitoring devices;
an output interface to the remote monitoring system;
a processor subsystem configured to, during the surgical procedure:
   - analyse the monitoring data to select a subset of the plurality of monitoring devices to be perceptually prioritized by the remote monitoring system, wherein said analysis uses a model arranged with surgical procedural knowledge and parameters and observability requirements for different states of the surgical procedure; and
   - send selection data indicative of said selection via the output interface to the remote monitoring system.

In a further aspect of the invention, a computer-implemented method is provided for being performed during a surgical procedure, wherein a plurality of monitoring devices is provided to monitor the surgical procedure, wherein a remote monitoring system is configured to render monitoring data provided by the plurality of monitoring devices to enable remote monitoring of the surgical procedure, wherein the method comprises:
- accessing the monitoring data provided by the plurality of monitoring devices;
- analysing the monitoring data to select a subset of the plurality of monitoring devices to be perceptually prioritized by the remote monitoring system, wherein said analysing comprises using a model arranged with surgical procedural knowledge and parameters and observability requirements for different states of the surgical procedure; and
- sending selection data indicative of said selection via the output interface to the remote monitoring system.

The above-identified two further aspects of the invention relate to the following. The monitoring control system may analyse the monitoring data to select a subset of the plurality of monitoring devices to be perceptually prioritized by the remote monitoring system. For that purpose, a model may be used which may be arranged with surgical procedural knowledge and parameters. For example, in case the model is a trained machine learning model, the training data of the machine learning model may include the surgical procedural knowledge and parameters. The surgical procedure knowledge may for example define the surgical steps, techniques, and protocols involved in the surgical procedure and the parameters may for example define possible positions of surgical instruments, possible complications, or anomalies, etc. The model may further include observability requirements which may characterize how the surgical procedure may be optimally observed, and in particular how a current state of the surgical procedure may be optimally observed. Such optimality may for example be defined in relation to surgical procedural objectives (which may be one or a successive and/or cumulative objectives over a surgical process), in that the observability requirements may indicate how the surgical procedure is best to be observed to reach such surgical procedural objective(s).

Using the model, one or more monitoring devices may be identified that provide a most valuable and informative view of the surgical procedure at a(ny) given time. In particular, the subset of monitoring devices may be selected to as much as possible meet the observability requirements identified by the model. For example, if the observability requirements indicate that an audio recording which captures the member(s) of staff is to be provided, the monitoring control system may select a microphone worn by the member(s) of staff for rendering. Another example is that if the observability requirements indicate that a camera recording is to be provided which provides an obstruction-free view of the surgical entry site, the monitoring control system may select a ceiling-mounted camera which is located above the patient and which provides the aforementioned obstruction-free view.

It is noted that in some examples, the model may directly generate a selection of monitoring devices from the monitoring data as input. In other words, the output of the model may be selection data. In other examples, the model may output observability requirements, and the subset of monitoring devices may be selected based on the observability requirements in another way, e.g., using heuristics or a different model.

It will be appreciated that the above-identified two further aspects of the inventions may be combined with any optional features described in this specification even if these optional features are described within the context of other aspects of the invention. In this respect, is noted that the model may, implicitly, for example as internal parameters or as hidden intermediate steps, or explicitly, determine a current state of the surgical procedure, determine one or more observability requirements for the current state of the surgical procedure, and based on the based on the one or more observability requirements, select a subset of the plurality of monitoring devices to be perceptual prioritized.

It will be appreciated by those skilled in the art that two or more of the above-mentioned embodiments, implementations, and/or optional aspects of the invention may be combined in any way deemed useful.

Modifications and variations of any of the systems, any of the computer-implemented methods and/or any of the computer programs, which correspond to the described modifications and variations of another one of said entities, can be carried out by a person skilled in the art on the basis of the present description.

### BRIEF DESCRIPTION OF THE DRAWINGS

These and other aspects of the invention will be apparent from and elucidated further with reference to the embodiments described by way of example in the following description and with reference to the accompanying drawings, in which:
Fig. 1 shows a surgical workspace comprising a surgical robot for performing one or more surgical steps on a patient, wherein the surgical workspace comprises a plurality of monitoring devices, and wherein a monitoring control system is provided to select a subset of the monitoring devices for rendering by a remote control system;
Fig. 2A shows a display area of the remote control system, with the display area simultaneously showing monitoring data of a plurality of monitoring devices;
Fig. 2B illustrates the highlighting of the monitoring data of a subset of monitoring devices by way of the outlines of respective windows being marked;
Fig. 2C illustrates the highlighting of the monitoring data of the subset of monitoring devices by way of the display layout being adjusted to increase the size of the respective windows showing the monitoring data of the subset of monitoring devices;
Fig. 2D illustrates the selective display of the monitoring data of the subset of monitoring devices;
Fig. 3 shows steps in which the monitoring control system is used to detect a deviation with respect to the planned surgical procedure and to address the deviation;
Fig. 4 shows a method of selecting monitoring devices for rendering; and
Fig. 5 shows a non-transitory computer-readable medium comprising data.

It should be noted that the figures are purely diagrammatic and not drawn to scale. In the figures, elements which correspond to elements already described may have the same reference numerals.

### List of reference numbers

The following list of reference numbers is provided for facilitating the interpretation of the drawings and shall not be construed as limiting the claims.
- 10, 12: camera
- 20: microphone
- 30: interventional imaging device
- 40: monitoring data
- 50: surgical workspace
- 60: surgical robot
- 70: patient
- 80: member of staff
- 90: network

- 100: monitoring control system
- 120: input interface
- 140: processor subsystem
- 160: output interface
- 162: selection data

- 200: remote control system
- 220: display
- 240: operator

- 300: display area showing monitoring data
- 301-303: monitoring data of selected monitoring devices
- 304-308: monitoring data of monitoring devices
- 310: display area with marking
- 320: display area with adjusted layout
- 330: display area with selective display of monitoring data

- 400: step in surgical procedure
- 402: continuing to following step in surgical procedure
- 410: determining if current step of surgical procedure deviates from planned surgical procedure
- 420: selecting subset of monitoring devices for rendering by remote control system
- 430: operator observes deviation through selected subset of monitoring devices
- 440: operator instructs local staff to address deviation
- 450: local staff takes action to address deviation
- 460: if deviation is successfully addressed, return to previous state of rendering the monitoring devices
- 500: method of selecting monitoring devices for rendering
- 510: accessing monitoring data
- 520: determining current state of surgical procedure
- 530: determining observability requirements
- 540: selecting subset of monitoring devices for rendering
- 550: sending selection data to remote control system

- 600: non-transitory computer-readable medium
- 610: data representing computer program

### DETAILED DESCRIPTION OF EMBODIMENTS

The following refers to a surgical procedure in which a surgical robot performs one or more surgical steps on a patient, and in which the surgical robot is remotely controllable by a remote operator using a remote control system. In this context, the remote control system is not only used to control the surgical robot but also to access to monitoring data of a plurality of monitoring devices. Thus, the remote control system may serve as a remote monitoring system as well. It is important to note, however, that the use of a surgical robot during the surgical procedure is optional and that the following applies equally to a remote monitoring system that does not facilitate remote control of a surgical robot.

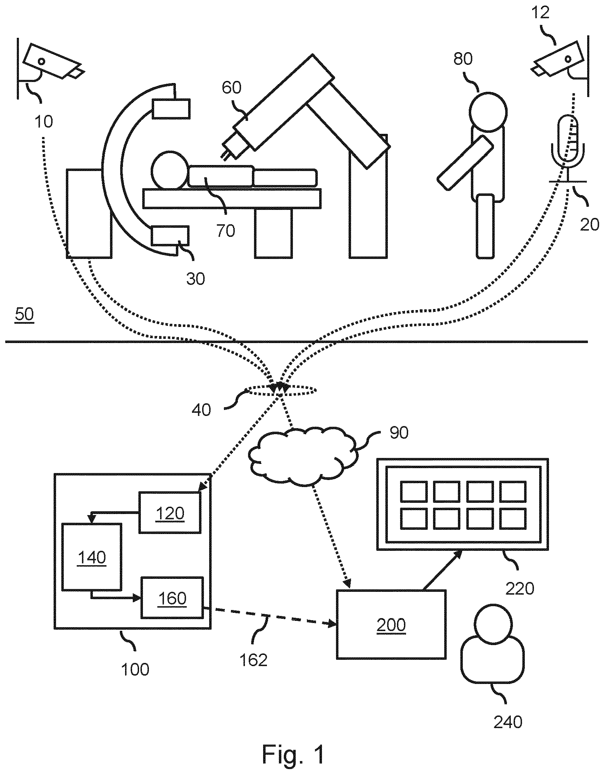

Fig. 1 shows a surgical workspace 50 comprising a surgical robot 60 for performing one or more surgical steps on a patient 70. The surgical workspace 50 is further shown to comprise an interventional imaging device 30 in form of a C-arm. Fig. 1 further shows a member of staff 80 being present in the surgical workspace. The member of staff may for example be a surgeon, an anaesthesiologist, a surgical assistant, etc. Although Fig. 1 only shows one member of staff 80 being present, typically several members of staff are present. The surgical workspace 50 is further shown to comprise a plurality of monitoring devices, which in this example include wall-mounted cameras 10-12 and a microphone 20.

Although not shown specifically in Fig. 1, the monitoring devices may also include other types of cameras, e.g., wearable cameras or ceiling-mounted cameras or cameras affixed to the surgical robot 60, other types of microphones, e.g., wearable microphones, and medical devices which generate medical data which may serve to monitor a medical state of the patient. For example, the C-arm 30 may serve as a monitoring device as the interventional imaging data generated by the C-arm may allow the patient and the surgical procedure to be monitored. Another example of a medical device serving as monitoring device may be a patient monitor. Yet other types of monitoring devices are sensors or components which monitor an operational state of an instrument or a surgical robot. For example, pressure sensors, force sensors, position sensors, orientation sensors or the like may be used to monitor the operational state of the surgical robot 60.

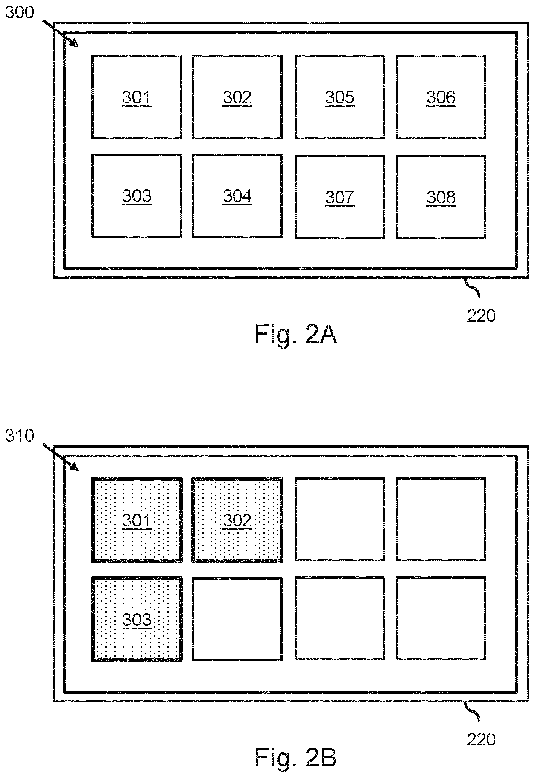

Fig. 1 further shows a remote control system 200 being provided. The remote control system 200 may be used by a remote operator 240 to control the surgical robot 60, for example using a joystick, mouse, or other user input device (not shown in Fig. 1). The remote control system 200 may comprise or be connected to a display 220 to provide visual feedback to the remote operator. For example, the display 220 may show a graphical user interface via which the surgical robot may be controlled. As will be elucidated with reference to Figs. 2A-2D, the graphical user interface may also allow the remote operator 240 to remotely monitor the surgical procedure, for example by showing camera feeds of the cameras 10, 12 and by showing interventional imaging data acquired by the C-arm 30. In general, the remote control system 200 may be configured to function as a remote monitoring system by providing the remote operator with access to the monitoring data 40 of the monitoring devices 10-30. Such monitoring data may include data which is rendered visually, e.g., by visualization on the display 220, but also data which is rendered auditory, e.g., by play-out via a loudspeaker. In some cases, the monitoring data may be rendered in another sensory perceptible manner, for example in a tactile or vestibular manner. It is noted that the remote control system 200 may access the monitoring data 40 of the monitoring devices 10-30 in any known manner, for example by data communication via a network 90.

Fig. 1 further shows a monitoring control system 100 which also has access to the monitoring data 40 of the monitoring devices. In the example of Fig. 1, the monitoring control system 100 is shown as a separate entity outside of the surgical workspace 50. However, the monitoring control system 100 may also be located inside of the surgical workspace 50, and may in general also be part of another entity. In a specific example, the monitoring control system 100 may be a subsystem of the remote control system 200.

The monitoring control system 100 is shown to comprise an input interface 120 to access the monitoring data 40 provided by the plurality of monitoring devices. Such an input interface may take various forms, such as a wired or wireless network interface for network-accessible monitoring devices. In other examples, the input interface 120 may be specific for a respective type of monitoring device. If there are several types of monitoring devices, several input interfaces may be provided, which together may form the input interface 120. For example, for a camera, a camera interface may be provided. In general, the input interface 120 may be or comprise a bus-type of interface, such as an USB interface, or a network interface, such as a wireless network interface based on Wi-Fi, Bluetooth, Zigbee, Matter, etc. or a wired network interface based on Ethernet or fibreoptics.

The monitoring control system 100 is further shown to comprise an output interface 160 to the remote control system 200. Such an output interface may take various forms, such as a wired or wireless network interface. In case the monitoring control system 100 is a subsystem of the remote control system 200, the output interface 160 may be an internal interface of the remote control system 200, such as a software-defined interface.

The monitoring control system 100 is further shown to comprise a processor subsystem 140 which may be configured to, during the surgical procedure, analyse the monitoring data 40 to determine a current state of the surgical procedure, determine one or more observability requirements for the current state of the surgical procedure based on observability data which is indicative of observability requirements for different states of the surgical procedure, based on the one or more observability requirements, select a subset of the plurality of monitoring devices to be perceptual prioritized by the remote control system 200, and send selection data 162 indicative of said selection via the output interface 160 to the remote control system. These and other aspects of the operation of the monitoring control system will be further elucidated in the following and with reference to Figs. 2A-4.

In general, the monitoring control system 100 may be embodied as, or in, a single device or apparatus. The device or apparatus may be a general-purpose device or apparatus, such as a workstation or a computer, but may also be an application-specific device or apparatus, such as a frontend device of a medical system. The device or apparatus may comprise one or more microprocessors which may represent the processor subsystem, and which may execute appropriate software. The software may have been downloaded and/or stored in a corresponding memory, e.g., a volatile memory such as RAM or a non-volatile memory such as Flash. Alternatively, functional units of the monitoring control system, e.g., the input interface, the output interface, and/or the processor subsystem, may be implemented in the device or apparatus in the form of programmable logic, e.g., as a Field-Programmable Gate Array (FPGA). In general, each functional unit of the monitoring control system 100 may be implemented in the form of a circuit. It is noted that the monitoring control system 100 may also be implemented in a distributed manner, e.g., involving different devices or apparatuses. For example, the distribution may be in accordance with a client-server model, e.g., using a server and workstation.

Fig. 2A shows a display area of the remote control system, with the display area simultaneously showing monitoring data 301-308 of a plurality of monitoring devices. For example, the monitoring data may include camera feeds, patient monitoring data, interventional images, measurement values of sensors, etc. In the example of Fig. 2A, each instance of monitoring data is shown in a separate window. It will be appreciated, however, that the monitoring data may be shown in any suitable manner, e.g., using different layouts.

The remote control system may be configured to, in response to the selection data generated by the monitoring control system, perceptually prioritize the monitoring data of the selected subset of monitoring devices in the rendering. Such perceptual prioritization may take different forms which may be dependent on the modality of the monitoring data, e.g., whether it is data to be visualized on a display or played-out via a loudspeaker, etc.

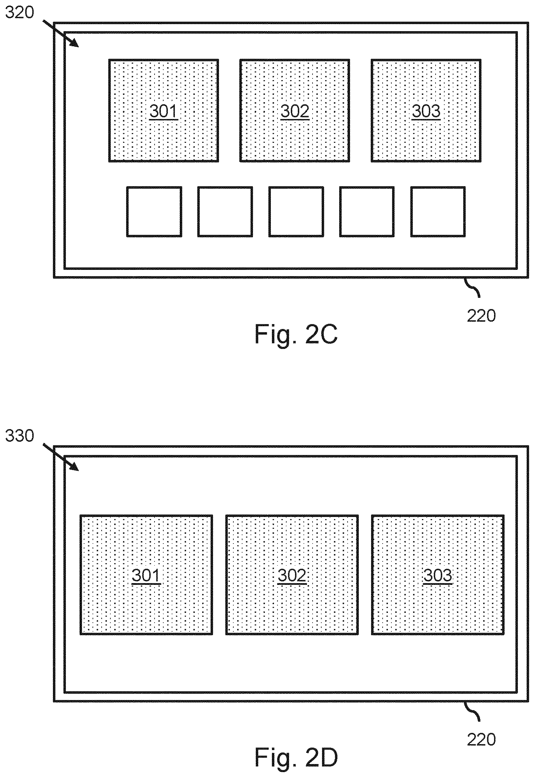

Figs. 2B-2D show examples of visual prioritization for visual monitoring data. In these examples, the selection data indicates that the monitoring data 301-303 is to be visually prioritized. Fig. 2B shows a display area 310 of the remote control system in which the windows 301-303 comprising data feeds from the selected subset of monitoring devices are highlighted by way of the outlines of respective windows being emphasized, thereby drawing the attention of the remote operator. Fig. 2C shows a display area 320 of the remote control system in which the windows 301-303 are increased in size. Finally, Fig. 2D shows a display area 330 of the remote control system in which the windows 301-303 are selectively displayed, in that other monitoring data is at least temporarily not displayed anymore.

It will be appreciated that there are various other ways to visually prioritize the monitoring data of a selected subset of monitoring devices and that the invention is not limited to any specific examples. For audio data, for example obtained from microphones or in form of synthesized speech which reads-out other types of monitoring data, perceptual prioritization may for example be obtained by selective play-out, e.g., with other audio sources being muted, or by increasing the play-out volume of a select monitoring device and/or lowering the play-out volume of other, non-selected monitoring devices.

The following illustrates different detailed embodiments and exemplary applications of the monitoring control system. However, these embodiments and applications should not be interpreted as limiting the scope of the invention as claimed.

One exemplary application is in a catheterization laboratory (`cath lab'). In such cath labs, remotely-controllable surgical robots may be used. However, the introduction of robotics into the cath lab in combination with remote control of the surgical robot by a remote surgeon introduces challenges to the way of working between, on the one hand, local staff in the cath lab, and on the other hand, the remote surgeon. In such settings, both parties may need to be well aware of what is happening and who is doing what in order to avoid possible accidents due to insufficient awareness of each other's actions. This may apply especially to situations where there is a deviation from a planned surgical procedure.

Examples of these deviations include but are not limited to:
- During a coronary intervention, an emergency bypass may be required when the surgeon is unable to recanalize an abruptly occluded vessel or a coronary perforation occurs.
- During stroke treatment, a sudden haemorrhage, or bleeding, in the brain may take place, e.g., because of a perforation or aneurysm rupture.
- During mechanical thrombectomy, when blood flow is restored, a haemorrhagic stroke may take place in the distal part of the now un-occluded vessel.

However, also other events may lead to deviations from the planned surgical procedure, such as mechanical defects in the surgical robot or changes occurring in the surgical workspace. Examples of these deviations include but are not limited to:
- A leak in a haemostatic valve
- New personal enters the surgical workspace
- The patient moves
- A new device is removed from its packaging
- The surgical robot has a malfunction
- The surgical robot unexpectedly moves
- The surgical robot has been equipped with an unexpected instrument, for example a microcatheter instead of the expected navigation catheter.

In case of such a deviation from the planned surgical procedure, or in case of any other occurrence in the surgical workspace, a lack of situational awareness of the remote surgeon may lead to serious workflow issues which may ultimately also endanger the patient. It is therefore desirable to enable the remote surgeon to obtain a sufficient degree of situational awareness. For that purpose, the monitoring control system as described elsewhere in this specification may be used to automatically switch between monitoring devices to provide optimal situational awareness to the remote surgeon, for example dependent on the current activity of the surgical robot, the current step within the surgical procedure, and local actions registered by the system. For example, if a certain step in the surgical procedure is being executed by a staff member, the monitoring control system may automatically provide a camera view that shows this surgical step being executed and the staff member executing the surgical step. Depending on the current activity of the remote surgeon, the camera feeds may appear in a main or secondary view on the remote control system used by the remote surgeon. Moreover, whenever it is determined that the surgical robot is not functioning as intended, e.g., a part is not where it should be, a relevant camera view may be displayed to the remote surgeon for quick assessment of the situation.

A specific example may involve the following. In a cath lab, an interventional imaging system may be provided, such as a Philips Azurion^{®} system. In addition, a remotely controlled surgical robot may be provided, which may be used for remote operations, for example for guidewire insertion. In the cath lab, wearable and/or stationary cameras may be provided, which may individually or jointly provide a 360-degree overview of the whole lab. Wearable and/or stationary microphones may be provided to capture conversations of the staff within the lab. The monitoring control system may be configured to process video, audio and digital data captured from the cath lab and to convert said data to comprehensive chunks by using data analysis techniques such as computer vision and natural language processing techniques and by using procedural knowledge. Based thereon, the monitoring control system may determine the current step in the surgical procedure.

In some examples, one or more machine learning models may be provided to recognize surgical steps and to determine their observability requirements or to directly select monitoring devices. An example of such a model is a deep learning model that may be trained, for example in a supervised manner, on video recordings of surgical procedures (for example, recordings used for training purposes and/or recordings made using monitoring devices as described in this specification) and surgery procedure textbooks. Accompanying audio recordings may be passed through a speech recognition algorithm to convert the audio recordings to text, e.g., to obtain a textural labelling for the video data. The model may then be trained on the labelled video data and textual data describing surgical procedure steps, e.g., as may be retrieved from textbooks. After training, the model may be used to recognize surgical procedure steps from the feeds of one or more monitoring devices, e.g., from one or more camera feeds. In a specific example, the model may be further trained to recognise deviations in the surgical procedure, for example by including video recordings of events which represent deviations in the surgical procedure in the training set and by labelling the video recordings accordingly. In another example, the model may be extended to detect a status of the surgical robot, for example by adding labelled log data (e.g., with system/error states) from the surgical robot to the training set. Alternatively, or additionally, the model may be trained to recognise a current surgical procedure step by using posture, gesture, and surgical instrument recognition from video, such as the surgery phase recognition technique provide by Vision Surgery AI (https://vision-surgery.ai). The model may be applied to or compared with the live video, audio, and digital data from the lab, for example to detect if there is a deviation from a current pre-planned surgical pathway.

In some examples, a second model, such as a second deep learning model, may be used to determine which monitoring devices to select for rendering based on the output of the model described in the preceding paragraph. For example, the second model may be trained in a supervised manner on selection data describing reference selections of audio, video, and system information feeds and on a labelling of the current surgical procedure step and/or a deviation, as may be provided by the first deep learning model. These reference selections may be obtained from recorded manual selections by a remote surgeon, while the first deep learning model may be running during these recordings, so that both data sources can be combined at a later stage to train the second deep learning model.

In the remote control room, a workstation may be provided for the remote surgeon to control the remotely controllable surgical robot and for example the interventional imaging system. The workstation, which may implement the remote control system as described elsewhere in this specification, may show an overview of video and other data feeds from that cath lab, with the overview being optimized to the current activities in the cath lab. The remote surgeon may be provided with options to adjust the video and audio feeds whenever deemed necessary. To enhance awareness of the remote surgeon, additional data sources may be displayed, such as control screens of medical devices in the cath lab, x-ray images acquired by the interventional imaging system, etc.

The monitoring control system may in some embodiments access surgical planning data which is indicative of the planned surgical procedure to be conducted and determine the current state of the surgical procedure based on a comparison to the planned surgical procedure. Such surgical planning data may originate from several sources. For example, an overview of the planned procedure may be available from the electronic medical record of the patient, from a cath lab system, or any other source. For instance, the Philips Azurion system may be provided with an interventional workstation in the control room on which pre-procedure planning software may be run prior to the treatment. This planning may then be sent to the cath lab main monitor or any other display device to be displayed during the treatment, and may be provided to the monitoring control system.

The monitoring control system may, depending on a current state of the surgical procedure, select optimal camera and audio feeds, in that it may select monitoring devices to be rendered by the remote control system. In general, such selection may involve determining observability requirements for the current state of the surgical procedure, that is, for the current progress and/or status of the surgical procedure, and selecting the monitoring devices based on the observability requirements. Using the observability requirements, the monitoring control system may for example prioritize sources in several ways:
o Whenever a local staff member is addressing the remote operator directly, the audio feed(s) and video feed(s) capturing this staff member may be selected.
o Whenever a part of the procedure deviates from the planned surgical procedure, e.g., from the normal pre-planned surgical pathway, the monitoring control system may direct the remote operator to the source of this deviation by appropriately selecting monitoring devices for rendering. For example, in case of an accidental puncture, the remote surgeon may be provided with medica data from the patient monitor, a stop button to immediately stop any actions of the surgical robot, and may establish a video/audio connection with the local staff member(s) who should now take immediate actions.
o Whenever there is a deviation in the operational state of the surgical robot from the planned or expected operational state, for example when a part of the surgical robot is missing or needs replacement, or when the surgical robot needs to be (re)aligned, or when the surgical robot needs additional setup for another part of the surgical procedure, the monitoring control system may provide the remote operator with video feed(s) showing the surgical robot or a part thereof, with monitoring data obtained from sensors or components which monitor the operational state of the surgical robot, etc.

For example, a surgical robot may have sensors or other components to indicate instrument type, instrument position, force, leaks, slippage errors, etc. Such sensors and other components may provide data which may be indicative of the current state of the surgical procedure and may thereby serve as monitoring data. In some embodiments, the use of additional sensors by the surgical robot may be indicative of a deviation, for example that the surgical procedure is not going according to the pre-planned pathway.

In some embodiments, the monitoring control system may apply speech recognition and natural language processing to audio captures of conversations between members of staff, for example to determine the current state of the surgical procedure, and based thereon, select a subset of monitoring devices to be perceptually prioritized.

In some embodiments, the monitoring control system may determine the observability requirements using a rule-based system which may contain predetermined rules which map states of a surgical procedure, e.g., in terms of progress and/or status, to observability requirements. In other embodiments, a machine learning model or other artificial intelligence based technique may be used to determine the observability requirements, for example by having been trained on training data for that purpose. In general, observability requirements may be defined in relation to entities in the surgical workspace. For example, an observability requirement may define that any one of the following may be shown or otherwise rendered: a surgical target of the surgical procedure, a surgical access or entry point, an instrument used in the surgical procedure, a member of staff attending the surgical procedure, a patient, a surgical robot, and a medical device used during the surgical procedure. The monitoring control system may identify which monitoring data pertains to which entity by analysing the monitoring data, e.g., using computer vision or computer audition, or by analysing metadata which is associated with the monitoring data. For example, the metadata may indicate that certain medical data is patient monitoring data.

With reference to the remote control system, it is noted that the remote control system typically comprises or is connected to an output device to output selected audio and/or video feeds, such as a stationary display, a portable display, a head-mounted display, a loudspeaker, etc. The remote control system may also provide a control panel for controlling one or more medicals device in the surgical workspace, such as a patient monitor or an interventional imaging system, and/or a control panel or for controlling the surgical robot. The control panel(s) may be part of a graphical user interface provided by the remote control system. The remote control system may also comprise or be connected to an eye tracker which may allow the remote control system or the monitoring control system to determine whether certain monitoring data is relevant or if the monitoring data needs to be adjusted or switched to other monitoring data, for example which better shows the entity in the surgical workspace which the remote operator is directing his/her attention to.

In some embodiments, the plurality of monitoring devices may include monitoring devices which are remotely controllable by the monitoring control system. For example, a pan-tilt camera may be remotely controlled to pan and/or tilt. The monitoring control system may be configured to control a respective monitoring device based on the one or more observability requirements, e.g., to improve the observability of a particular step or situation or entity within the surgical workspace. In some embodiments, the monitoring control system may be configured to reconfigure monitoring devices to improve the observability of a particular step or situation or entity within the surgical workspace, for example by adjusting camera settings, microphone settings, image acquisition parameters of an interventional imaging device, operational parameters of the surgical robot, etc.

In some embodiments, the monitoring control system may send the monitoring data, e.g., of the subset of monitoring devices or of all monitoring devices, to the remote control system, instead of the remote control system receiving the monitoring data directly from the respective monitoring devices. This may allow the monitoring control system to process the monitoring data, e.g., to enhance the observability of certain events or entities, and provide the enhanced or otherwise processed monitoring data to the remote control system. In such embodiments, the monitoring control system may effectively function as a source or a relay or proxy of the monitoring data for the remote control system.

In some embodiments, the monitoring control system may be configured to receive the monitoring data from the plurality of monitoring devices, access a pre-planned robotically-driven procedural pathway, execute a model arranged with procedural knowledge and associated requirements/criteria for optimal remote monitoring of the pre-planned robotically-driven procedure, process the monitoring data and output a control signal for the remote monitoring system, wherein the control signal includes an identification of a selection of monitoring devices, and optionally a tuning of monitoring data from the selection of monitoring devices, for an optimal remote monitoring of the procedure. The control signal may represent the selection data as mentioned elsewhere in this specification. The control signal may then be sent to the remote monitoring system such that the remote monitoring system may perceptually prioritize the rendering data of the selected monitoring devices.

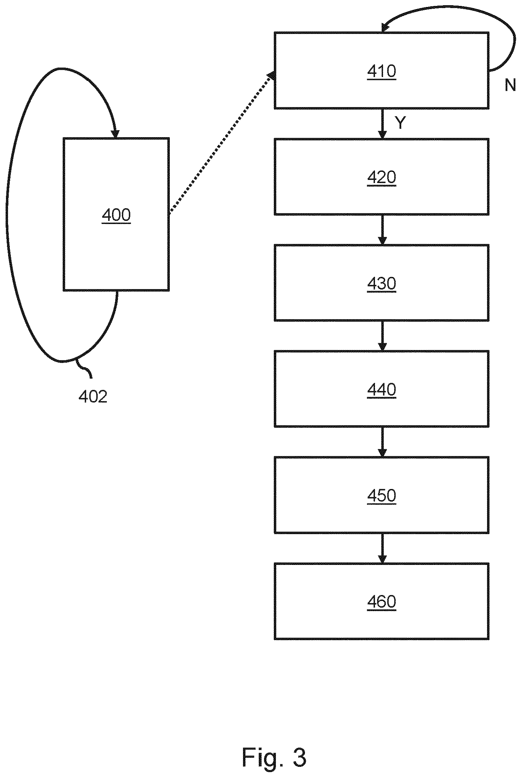

Fig. 3 illustrates a possible use case for the monitoring control system. For a current step 400 in the procedure, the monitoring control system may determine 410 if an operation if the current step of surgical procedure deviates from planned surgical procedure. If yes (Y), a subset of monitoring devices may be selected 420 which allows the deviation to be observed. Accordingly, the operator may observe 430 the deviation through the selected subset of monitoring devices and instruct 440 local staff to address the deviation. In turn, local staff may take action 450 to address the deviation. If the deviation is successfully addressed, the monitoring control system may revert 460 to a previously selected set of monitoring devices, e.g., from before the deviation, or the remote control system itself may revert to a previous display layout or configuration. This may allow the remote operator to continue with a same or similar set of monitoring devices as before the deviation occurred.

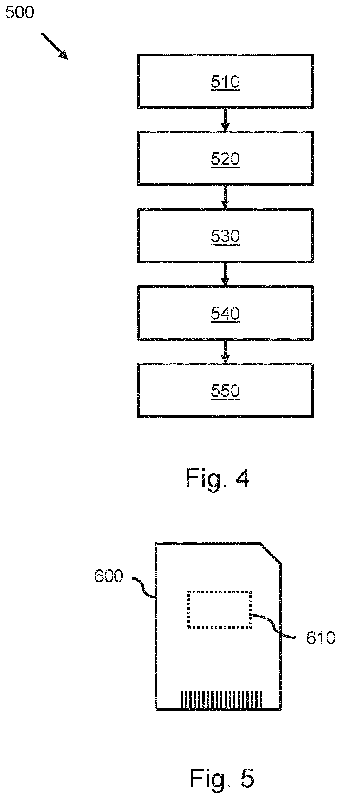

Fig. 4 shows a method 500 for being performed during a surgical procedure. The method 500 may correspond to an operation of the monitoring control system 100 of Fig. 1. However, this is not a limitation, in that the computer-implemented method 500 may also be performed using another system, apparatus or device. The method 500 is shown to comprise, in a step titled "ACCESSING MONITORING DATA", accessing 510 the monitoring data provided by the plurality of monitoring devices, in a step titled "DETERMINING CURRENT STATE OF SURGICAL PROCEDURE", analysing 520 the monitoring data to determine a current state of the surgical procedure, in a step titled "DETERMINING OBSERVABILITY REQUIREMENTS", determining 530 one or more observability requirements for the current state of the surgical procedure based on observability data which is indicative of observability requirements for different states of the surgical procedure, in a step titled "SELECTING SUBSET OF MONITORING DEVICES FOR RENDERING", based on the one or more observability requirements, selecting 540 a subset of the plurality of monitoring devices to be perceptual prioritized by the remote monitoring system, and in a step titled "SENDING SELECTION DATA TO REMOTE CONTROL SYSTEM", sending 550 selection data indicative of said selection via the output interface to the remote monitoring system.

It will be appreciated that the steps of the method 500 may be performed in any suitable order, e.g., consecutively, simultaneously, or a combination thereof, subject to, where applicable, a particular order being necessitated, e.g., by input/output relations.

The method may be implemented on a computer as a computer implemented method, as dedicated hardware, or as a combination of both. As also illustrated in Fig. 5, instructions for the computer, e.g., executable code, may be stored on a computer readable medium 600, e.g., in the form of a series 610 of machine-readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, etc. Fig. 5 shows a memory device 600.

Examples, embodiments or optional features, whether indicated as non-limiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of' when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A monitoring control system (100) for use in a surgical procedure, wherein a plurality of monitoring devices (10-30) is provided to monitor the surgical procedure, wherein a remote monitoring system (200) is configured to render monitoring data provided by the plurality of monitoring devices to enable remote monitoring of the surgical procedure, wherein the monitoring control system comprises:
- an input interface (120) for accessing the monitoring data (40) provided by the plurality of monitoring devices;
- an output interface (160) to the remote monitoring system;
- a processor subsystem (140) configured to, during the surgical procedure:
- analyse the monitoring data to determine a current state of the surgical procedure;
- determine one or more observability requirements for the current state of the surgical procedure based on observability data which is indicative of observability requirements for different states of the surgical procedure;
- based on the one or more observability requirements, select a subset (301-303) of the plurality of monitoring devices to be perceptual prioritized by the remote monitoring system; and
- send selection data (162) indicative of said selection via the output interface to the remote monitoring system.

2. The system (100) according to claim 1, wherein the processor subsystem (140) is configured to:
- access surgical planning data which is indicative of a planned surgical procedure to be conducted;
- determine the current state of the surgical procedure based on a comparison to the planned surgical procedure.

3. The system (100) according to claim 2, wherein the processor subsystem (140) is configured to:
- based on the comparison, determine whether the current state represents a deviation from the planned surgical procedure; and
- if the deviation is determined, select the subset (301-303) to include at least one monitoring device through which the deviation is remotely observable.

4. The system (100) according to claim 3, wherein the processor subsystem (140) is configured to:
- analyse the monitoring data (40) to identify an entity which has caused, contributed to, or is otherwise associated with the deviation; and
- select the subset to include at least one monitoring device through which the entity is remotely observable.

5. The system (100) according to claim 4, wherein the entity is at least one of:
- a surgical target of the surgical procedure,
- a surgical access point,
- an instrument used in the surgical procedure,
- a member of staff attending the surgical procedure (80),
- a patient (70),
- a surgical robot (60), and
- a medical device (30) used during the surgical procedure.

6. The system (100) according to any one of claims 1 to 5, wherein the plurality of monitoring devices comprises at least one of: an audio-visual recording device, such as a camera (10, 12) or a microphone (20), a sensor or component configured to monitor an operational state of an instrument or a surgical robot (60), and a medical device, such as a patient monitor or an interventional imaging system (30).

7. The system (100) according to any one of claims 1 to 6, wherein the processor subsystem (140) is configured to:
- based on the current state of the surgical procedure, predict a following step of the surgical procedure; and
- based on observability data, select the subset to include at least one monitoring device through which the following step is remotely observable.

8. The system (100) according to any one of claims 1 to 7, wherein the processor subsystem (140) is configured to determine the current state of the surgical procedure by determining a current step in the surgical procedure, for example by identifying a surgical event in the monitoring data (40), for example using computer vision or computer audition.

9. The system (100) according to any one of claims 1 to 8, wherein the remote monitoring system (200) is configured to enable a remote user of the remote monitoring system to communicate with a member of staff attending the surgical procedure, wherein the processor subsystem (140) is configured to select the subset to include at least one monitoring device through which the member of surgical staff is perceptually perceivable, for example visually and/or auditory.

10. The system (100) according to any one of claims 1 to 9, wherein the remote monitoring system (200) comprises or is connected to an eye-tracker to eye-track a remote user of the remote monitoring system, and wherein the processor subsystem (140) is further configured to:
- access eye tracking data from the eye-tracker to determine which monitoring data is observed by the remote user; and
- adjust the selection data based on the eye tracking data.

11. The system (100) according to any one of claims 1 to 10, wherein the remote monitoring system (200) is configured to perceptually prioritize the monitoring data of the subset of the plurality of monitoring devices by selectively rendering or perceptually highlighting the monitoring data of the subset of the plurality of monitoring devices.

12. The system (100) according to any one of claims 1 to 11, wherein a surgical robot (60) performs one or more surgical steps during the surgical procedure, and wherein the remote monitoring system is a remote control system (200) to remotely control the surgical robot.

13. A monitoring control system (100) for use in a surgical procedure, wherein a plurality of monitoring devices (10-30) is provided to monitor the surgical procedure, wherein a remote monitoring system (200) is configured to render monitoring data provided by the plurality of monitoring devices to enable remote monitoring of the surgical procedure, wherein the monitoring control system comprises:
- an input interface (120) for accessing the monitoring data (40) provided by the plurality of monitoring devices;
- an output interface (160) to the remote monitoring system;
- a processor subsystem (140) configured to, during the surgical procedure:
- analyse the monitoring data to select a subset (301-303) of the plurality of monitoring devices to be perceptually prioritized by the remote monitoring system, wherein said analysis uses a model arranged with surgical procedural knowledge and parameters and observability requirements for different states of the surgical procedure; and
- send selection data (162) indicative of said selection via the output interface to the remote monitoring system.

14. A transitory or non-transitory computer-readable medium (600) comprising data (610) representing a computer program, the computer program comprising instructions for causing a processor system to perform a method (500) for being performed during a surgical procedure, wherein a plurality of monitoring devices is provided to monitor the surgical procedure, wherein a remote monitoring system is configured to render monitoring data provided by the plurality of monitoring devices to enable remote monitoring of the surgical procedure, wherein the method comprises:
- accessing (510) the monitoring data provided by the plurality of monitoring devices;
- analysing (520) the monitoring data to determine a current state of the surgical procedure;
- determining (530) one or more observability requirements for the current state of the surgical procedure based on observability data which is indicative of observability requirements for different states of the surgical procedure;
- based on the one or more observability requirements, selecting (540) a subset of the plurality of monitoring devices to be perceptual prioritized by the remote monitoring system; and
- sending (550) selection data indicative of said selection via the output interface to the remote monitoring system.

15. A transitory or non-transitory computer-readable medium (600) comprising data (610) representing a computer program, the computer program comprising instructions for causing a processor system to perform a method (500) for being performed during a surgical procedure, wherein a plurality of monitoring devices is provided to monitor the surgical procedure, wherein a remote monitoring system is configured to render monitoring data provided by the plurality of monitoring devices to enable remote monitoring of the surgical procedure, wherein the method comprises:
- accessing (510) the monitoring data provided by the plurality of monitoring devices;
- analysing (520) the monitoring data to select (530) a subset of the plurality of monitoring devices to be perceptually prioritized by the remote monitoring system, wherein said analysing comprises using a model arranged with surgical procedural knowledge and parameters and observability requirements for different states of the surgical procedure; and
- sending (540) selection data indicative of said selection via the output interface to the remote monitoring system.
